(19) Europäisches Patentamt
European Patent Office
Office européen des brevets

(11) Numéro de publication: **0 058 146**
**B1**

# (12) FASCICULE DE BREVET EUROPEEN

(45) Date de publication du fascicule du brevet: **27.06.84**

(51) Int. Cl.³: **C 07 D 295/08**, A 61 K 31/495

(21) Numéro de dépôt: **82870006.2**

(22) Date de dépôt: **05.02.82**

(54) **Nouveaux acides 2-(4-(diphénylméthyl)-1-pipérazinyl)-acétiques et leurs amides, leurs procédés de préparation et compositions thérapeutiques.**

(30) Priorité: **06.02.81 GB 8103768**
**08.04.81 GB 8110990**

(43) Date de publication de la demande:
**18.08.82 Bulletin 82/33**

(45) Mention de la délivrance du brevet:
**27.06.84 Bulletin 84/26**

(84) Etats contractants désignés:
**AT BE CH DE FR GB IT LI LU NL SE**

(56) Documents cités:
**BE - A - 763 609**
**FR - A - 2 082 168**
**FR - A - 2 173 982**
**US - A - 3 244 718**

(73) Titulaire: **U C B, S.A., 326, Avenue Louise, B-1050 Bruxelles (BE)**

(72) Inventeur: **Baltes, Eugène, 2, Avenue des Chênes, B-1640 Rhode-St.-Genese (BE)**
Inventeur: **de Lannoy, Jean, 47, Avenue Alexandre Bertrand, B-1190 Bruxelles (BE)**
Inventeur: **Rodriguez, Ludovic, 12, Clos Dandoy, B-1180 Bruxelles (BE)**

(74) Mandataire: **Vanderborght, Henri et al, U C B, S.A. Département D.T.B. 33, rue d'Anderlecht, B-1620 Drogenbos (BE)**

EP 0 058 146 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

**Description**

La présente invention se rapporte à de nouveaux acides 1-[4-(diphénylméthyl)-1-pipérazinyl]-acétiques, à leurs amides, à leurs sels non toxiques pharmaceutiquement acceptables ainsi qu'à des procédés pour les préparer. Elle concerne également les compositions thérapeutiques renfermant lesdits composés.

Les nouveaux composés de la présente invention répondent à la formule générale

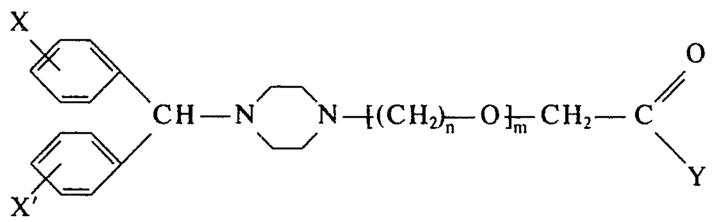

(I)

dans laquelle

Y est un groupe $-OH$ ou un groupe $-NH_2$,
X et X', pris isolément, représentent un atome d'hydrogéne, un atome d'halogene, un radical alkoxy inférieur linéaire ou ramifié ou un radical trifluorométhyle,
m est égal à 1 ou 2,
n est égal à 1 ou 2, de préférence 2,

ainsi que leurs sels non toxiques pharmaceutiquement acceptables.

Par l'expression »alkoxy inférieur« on entend des restes d'alcools aliphatiques à chaîne droite ou ramifiée renfermant de 1 à 4 atomes de carbone, tels que méthoxy, éthoxy, propoxy, etc. L'atome d'halogène est de préférence un atome de chlore ou de fluor.

Dans le cadre de la présente invention, on entend par »sels non toxiques pharmaceutiquement acceptables« non seulement les sels d'addition des acides et des amides de formule I avec des acides pharmaceutiquement acceptables comme l'acide acétique, l'acide citrique, l'acide succinique, l'acide ascorbique, l'acide chlorhydrique, l'acide bromhydrique, l'acide sulfurique ou l'acide phosphorique, mais également les sels pharmaceutiquement acceptables des acides de formule I comme les sels de métaux (par exemple sodium ou potassium), les sels d'ammonium, les sels d'amines ou d'acides aminés.

Ces sels pharmaceutiquement acceptables peuvent être préparés à partir des composés de formule I par des méthodes connues en soi.

Les composés préférés conformes à l'invention sont:

- l'acide 2-[2-[4-[(4-chlorophényl)phénylméthyl]-1-pipéranzinyl]éthoxy]-acétique et son dichlorhydrate,
- le sel de potassium de l'acide 2-[2-[4-[(4-chlorophényl)phénylméthyl]-1-pipérazinyl]éthoxy]-acétique,
- l'acide 2-[2-[4-(diphénylméthyl)-1-pipérazinyl]éthoxy]-acétique et son dichlorhydrate,
- l'acide 2-[2-[4-[(4-fluorophényl)phénylméthyl]-1-pipérazinyl]éthoxy]-acétique et son hydrate.

Les composés de formule I possèdent des propriétés pharmacologiques intéressantes; ils s'avèrent notamment utiles comme agents antiallergiques, antihistaminiques, bronchodilatateurs et antispasmodiques.

En plus, ils se caractérisent par le fait que leurs effets secondaires de stimulation ou de dépression du système nerveux central, couramment observés avec les antihistaminiques usuels, sont minimes. En outre, ils présentent de l'intérêt en tant qu'anesthésiques et antiinflammatoires et ils s'avèrent actifs dans l'insuffisance cérébrale ou cardiovasculaire.

Dans un article de H. B. WRIGHT et D. L. MARTIN (J. med. Chem. 11, (1968), 390–391) on étudie l'activité du 2-[4-[(4-chlorophényl)phénylméthyl]-1-pipérazinyl]-acétamide (Formule I: $Y=NH_2$; $X=p-Cl$; $X'=H$; $m=O$) comme agent hypocholestérémiant, mais les résultats obtenus étaient peu encourageants.

Pair ailleurs, dans le brevet belge 763.609, on décrit le 2-[4-(diphénylméthyl)-1-pipérazinyl]-acétamide (Formule I: $Y=NH_2$; $X=X'=H$; $m=O$) comme possèdant une activité diurétique. Il décrit également des acétamides similaires, mais dont l'atome d'azote du groupe amide est substitué et qui possèdent diverses autres propriétés pharmacologiques.

Des essais pharmacologiques effectués par la demanderesse ont montré que l'activité antiallergique, antispasmodique et antihistaminique de ces acétamides, ainsi que celle des acides correspondants était peu intéressante. (Voir B. Pharmacologie).

A. Procédés de préparation.

I. Les amides de formule I ($Y = -NH_2$) peuvent être préparés par diverses méthodes, à savoir

I. 1. en faisant réagir une 1-(diphénylméthyl)-pipérazine de formule II avec un oméga-halogéno-acétamide de formule III, selon l'équation

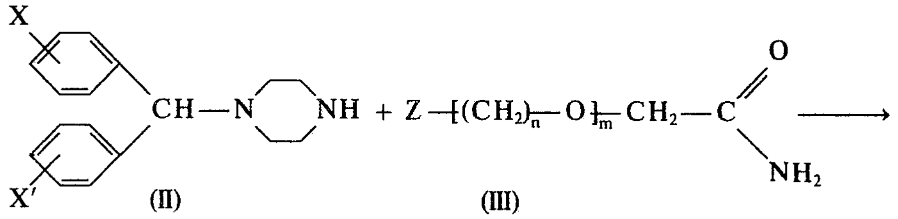

(II) (III)

(I) avec $Y = -NH_2$

dans lesquelles X, X', m et n ont la signification donnée plus haut et Z represénte un atome d'halogène.

Cette réaction est généralement effectuée par chauffage, entre 80 et 150°C, pendant plusieurs heures, dans un solvant inerte choisi parmi des alcools aliphatiques, des cétones aliphatiques (p. ex. la méthyléthylcétone), des hydrocarbures aromatiques (p. ex. le toluène ou le xylène) et en présence d'un accepteur d'acide tel qu'une base organique tertiaire (p. ex. la triéthylamine) ou une base minérale (p. ex. le carbonate de sodium).

I. 2. en faisant réagir un sel de métal alcalin d'un oméga-[4-(diphénylméthyl)-1-pipérazinyl]-alcanol de formule IV avec un 2-halogénoacétamide de formule V, selon l'équation

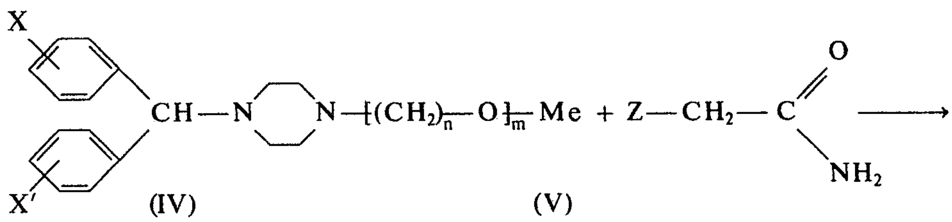

(IV) (V)

(I) avec $Y = -NH_2$

dans lesquelles X, X', m et n ont la signification donnée plus haut, Z représente un atome d'halogène et Me un métal alcalin.

La réaction entre le sel de métal alcalin de formule IV et l'acétamide halogéné de formule V est effectuée dans un solvant inerte (p. ex. le toluène, le xylène ou le diméthylformamide), à une température comprise entre 0°C et la température de reflux du mélange réactionnel.

Le sel de métal alcalin de formule IV utilisé dans cette réaction est préparé in situ en faisant réagir l'oméga-[4-(diphénylméthyl)-1-pipérazinyl]-alcanol approprié avec un hydrure de métal alcalin, généralement l'hydrure de sodium, dans un solvant inerte tel que le toluène, le xylène ou le diméthylformamide.

Quant à la préparation des alcools de formule IV (Me = H), elle est décrite dans le brevet américain 2.899.436 au nom de la demanderesse.

I. 3. en faisant réagir l'ammoniac avec un dérivé fonctionnel d'un acide 2-[4-(diphénylméthyl)-1-pipérazinyl]-acétique, à savoir un halogénure ou un ester d'alkyle inférieur de formule VII, selon l'équation

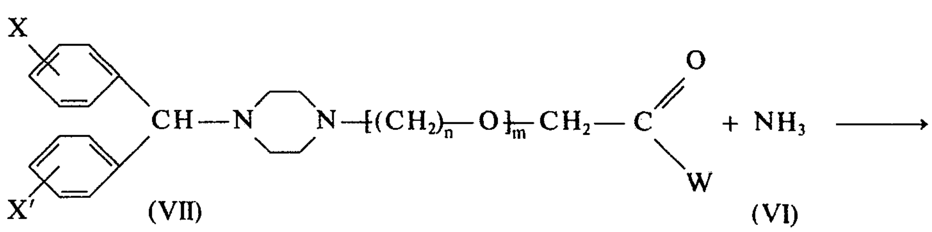

(VII) (VI)

(I) avec $Y = -NH_2$

dans lesquelles X, X', m et n ont la signification donnée plus haut et W représente un atome

d'halogène ou un radical −OR′ dans lequel R′ représente un radical alkyle inférieur.

L'atome d'halogène peut par exemple être du chlore ou du brome et le radical alkyle un radical méthyle ou éthyle.

Dans le cas où W represénte un atome d'halogène, on commence par préparer un acide de formule I (Y = OH) par la méthode II décrite ci-après et on le transforme en son halogénure correspondant, selon les méthodes classiques pour préparer ce genre de composé. Ensuite, on fait réagir l'halogénure d'acide obtenu avec l'ammoniac dans un solvant inerte.

Dans le cas où W représente un radical −OR′, on commence par prépare un ester de formule VII par l'une des méthodes décrites ci-après.

Ensuite, on fait réagir cet ester avec l'ammoniac dans un solvant inerte, à une température comprise entre 0°C et la température ambiante. Cette réaction peut éventuellement être effectuée en présence d'un catalyseur tel que méthylate de sodium.

II. Les acides de formule I dans laquelle Y = OH sont préparés par hydrolyse, en milieu basique, d'un dérivé fonctionnel d'un acide 2-[4-(diphénylméthyl)-1-pipérazinyl]-acétique, à savoir un amide ou un ester d'alkyle inférieur de formule

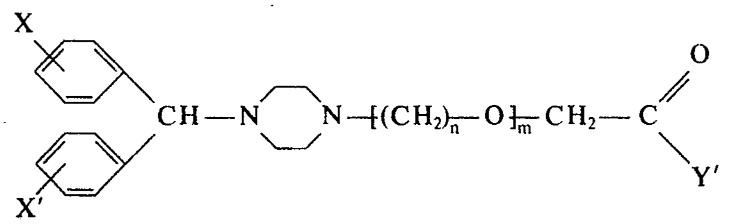

(VIII)

dans laquelle X, X′, m et n ont la signification donnée plus haut et Y′ représente un groupe $-NH_2$ ou un groupe −OR′ dans lequel R′ représente un radicaal alkyle inférieur (par exemple un radical méthyle ou éthyle).

Cette hydrolyse est effectuée au moyen d'une base minérale telle que l'hydroxyde de sodium ou de potassium, en milieu aqueux ou hydroalcoolique (méthanol, éthanol, etc.), à une température comprise entre 20°C et la température de reflux du mélange réactionnel.

Les amides de formule VIII dans laquelle Y′ = $-NH_2$ peuvent être préparés par l'une des méthodes I.1 à I.3 décrites plus haut.

Quant aux esters de formule VII dans laquelle W = −OR′ et les esters de formule VIII dans laquelle Y′ = −OR′, ils peuvent être préparés par diverses méthodes, paar exemple:

a) en faisant réagir une 1-(diphénylméthyl)-pipérazine de formule II avec un oméga-halogénoacétate d'alkyle inférieur de formule IX, selon l'équation

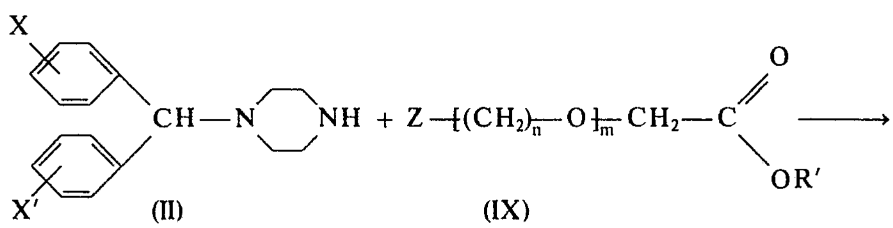

(II) (IX)

(VII) avec W = −OR′ ou
(VIII) avec Y′ = −OR′

dans lesquelles X, X′, m et n ont la signification donnée plus haut, R′ représente un radical alkyle inférieur et Z un atome d'halogène. Ainsi, R′ est par exemple un radical méthyle ou éthyle et Z un atome de chlore ou de brome.

Cette réaction est généralement effectuée par chauffage, entre 80 et 150°C, pendant plusieurs heures, dans un solvant inerte, tels que le benzène, le toluène ou le xylène et en présence d'un accepteur d'acide tel qu'une base organique tertiaire (par exemple la triéthylamine) ou une base minérale (par exemple le carbonate de sodium).

b) en faisant réagir un sel de métal alcalin d'un oméga-[4-(diphénylméthyl)-1-pipérazinyl]-alcanol de formule IV avec un ester d'alkyle inférieur d'un acide 2-halogénoacétique de formule X, selon l'équation

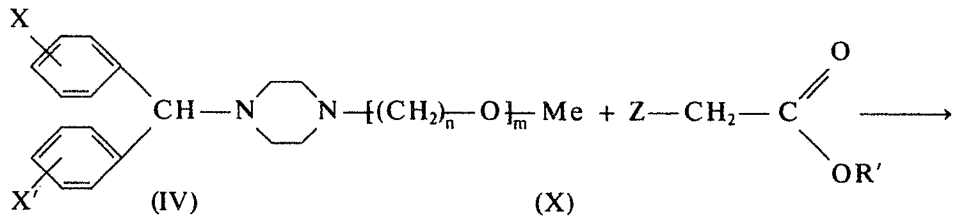

(IV) (X)

(VII) avec W = OR' ou
(VIII) avec Y' = OR'

dans lesquelles R', X, X', m et n ont la signification donnée plus haut Z uprésente un atome d'halogène et Me un métal alcalin.
La réaction entre le sel de métal alcalin de formule IV et l'ester halogéné de formule X est effectuée dans un solvant inerte, à une température comprise entre 0°C et la température de reflux du mélange réactionnel.

Les exemples qui suivent illustrent l'invention sans la limiter.

Exemple 1. Préparation des amides de formule I (Y = $-NH_2$)

1.1. Dichlorhydrate de 2-[2-[4-(diphénylméthyl)-1-pipérazinyl]éthoxy]-acétamide. (méthode I.1).

On chauffe pendant 4 heures, entre 90 et 120°C, un mélange de 37,8 g (0,15 mole) de 1-(diphénylméthyl)-pipérazine, de 27,5 g (0,2 mole) de 2-(2-chloréthoxy)-acétamide et de 26,5 g de carbonate de sodium anhydre dans 120 ml de xylène. On ajoute ensuite 120 ml de benzène, on filtre le précipité formé et on extrait la couche organique avec une solution diluée d'acide chlorhydrique (30 ml d'acide chlorhydrique concentré + 100 ml d'eau). Après l'addition de 40 ml d'une solution concentrée d'hydroxyde de sodium et extraction au benzène, on lave la solution benzénique à l'eau, on la sèche sur du carbonate de sodium anhydre et on évapore le benzène jusqu'à siccité. On triture lle résidu d'évaporation avec de l'éther éthylique et on laisse cristalliser. On obtient le 2-[2-[4-(diphénylméthyl)-1-pipérazinyl]éthoxy]-acétamide avec un rendement de 73%.P.F.: 119—120°C.
Le dichlorhydrate, préparé dans l'éthanol, fond à 230°C avec décomposition.

Analyse pour $C_{21}H_{27}N_3O_2 \cdot 2\ HCl$ en %

| | | | | |
|---|---|---|---|---|
| calculé: | C 59,15 | H 6,85 | N 9,85 | $Cl^-$ 16,63 |
| trouvé: | C 58,99 | H 6,80 | N 9,79 | $Cl^-$ 16,46 |

Les produits suivants ont été préparés par méthode décrite cidessus:

— 2-[2-[4-[(4-chlorophényl)phénylméthyl]-1-pipérazinyl]éthoxy]-acétamide.

Rendement: 47 % P.F.: 111—112°C (éthanol)

Analyse pour $C_{21}H_{26}ClN_3O_2$ en %

| | | | | |
|---|---|---|---|---|
| calculé: | C 65,02 | H 6,71 | N 10,83 | Cl 9,14 |
| trouvé: | C 64,59 | H 7,00 | N 10,82 | Cl 9,54 |

— 2-[2-[2-[4-(diphénylméthyl)-1-pipérazinyl]éthoxy]éthoxy]-acétamide.
Le produit, obtenu à l'état brut avec un rendement pratiquement quantitatif, a été utilisé tel quel, sans autre purification, pour préparer l'acide correspondant (exemple 2.2).
— 2-[2-[2-[4-[(4-chlorophényl)phénylméthyl]-1-pipérazinyl]éthoxy]éthoxy]-acétamide.
Le produit, obtenu à l'état brut avec un rendement pratiquement quantitatif, a été utilisé tel quel, sans autre purification, pour préparer l'acide correspondant (exemple 2.2).
— 2-[2-[4-[(4-fluorophényl)phénylméthyl]-1-pipérazinyl]éthoxy]-acétamide.

Rendement: 54,7 % P.F.: 105—107°C (acétonitrile)

Analyse pour $C_{21}H_{26}FN_3O_2$ en %

| | | | |
|---|---|---|---|
| calculé: | C 67,90 | H 7,09 | N 11,31 |
| trouvé: | C 68,3 | H 7,40 | N 11,21 |

– 2-[2-[4-[(2-chlorophényl)phénylméthyl]-1-pipérazinyl]éthoxy]-acétamide.

Rendement: 57 % P.F.: 129–130°C (benzène)

Analyse pour $C_{21}H_{26}ClN_3O_2$ en %

| | | | | |
|---|---|---|---|---|
| calculé: | C 65,0 | H 6,75 | N 10,8 | Cl 9,4 |
| trouvé: | C 66,3 | H 7,0 | N 10,6 | Cl 9,7 |

Le dichlorhydrate de cet amide a également été préparé; il s'avère qu'il contient du monochlorhydrate.

P.F.: 218–220°C (alcool isopropylique)

Analyse pour $C_{21}H_{26}ClN_3O_2 \cdot 2HCl$ en %

| | | | | | |
|---|---|---|---|---|---|
| calculé: | C 54,72 | H 6,12 | N 9,11 | $Cl^-$ 15,38 | $Cl^{tot.}$ 23,08 |
| trouvé: | C 55,69 | H 6,52 | N 9,20 | $Cl^-$ 11,86 | $Cl^{tot.}$ 20,27 |

– dichlorhydrate de 2-[2-[4-[(4-méthoxyphényl)phénylméthyl]-1-pipérazinyl]éthoxy]-acétamide.

Rendement: 20 % P.F.: 175–176°C (acétonitrile)

Analyse pour $C_{22}H_{29}N_3O_3 \cdot 2HCl$ en %

| | | | | |
|---|---|---|---|---|
| calculé: | C 57,8 | H 6,8 | N 9,2 | $Cl^-$ 15,5 |
| trouvé: | C 57,8 | H 7,2 | N 9,5 | $Cl^-$ 15,9 |

– 2-[2-[2-[4-[4-(trifluorométhyl)phényl]phénylméthyl]-1-pipérazinyl]éthoxy]éthoxy]-acétamide.
– 2-[2-[2-[4-[bis(4-fluorophényl)méthyl]-1-pipérazinyl]éthoxy]éthoxy]-acétamide.

1.2. Dichlorhydrate de 2-[2-[2-[4-(diphénylméthyl)-1-pipérazinyl]éthoxy]éthoxy]-acétamide. (méthode I. 2.)

On ajoute 24,2 g (0,53 mole) d'hydrure de sodium à une solution de 172,9 g (0,508 mole) de 2-[2-[4-(diphénylméthyl)-1-pipérazinyl]éthoxy]-éthanol dans 180 ml de diméthylformamide. Une fois l'addition terminée, on chauffe le mélange à 40°C pendant 30 minutes. Après refroidissement, on y ajoute en 10 minutes 60 g (0,624 mole) de 2-chloracétamide. La température du mélange réactionnel monte à 40°C et on maintient cette température encore pendant 30 minutes. On refroidit, on ajoute 30 ml d'eau et on évapore à siccité. On met le résidu en suspension dans l litre d'eau et on extrait la suspension obtenue au benzène. La phase organique est séchée sur du carbonate de potassium, puis évaporée. Le résidu est purifié par chromatographie sur colonne de silice (éluant: chloroformeéthanol, 95 : 5). Le produit obtenu est dissous dans 45 ml d'éthanol auxquels on ajoute 24 ml d'une solution éthanolique d'acide chlorhydrique 5,1 N.
On obtient 19 g de dichlorhydrate de 2-[2-[2-[4-(diphénylméthyl)-1-pipérazinyl]éthoxy]éthoxy]-acétamide avec un rendement de 8 %. P.F.: 196–197°C (acétonitrile).

Analyse pour $C_{23}H_{31}N_3O_3 \cdot 2HCl$ en %

| | | | | |
|---|---|---|---|---|
| calculé: | C 58,72 | H 7,07 | N 8,93 | $Cl^-$ 15,07 |
| trouvé: | C 58,29 | H 6,83 | N 8,44 | $Cl^-$ 15,01 |

Le produit suivant a été préparé par la méthode décrite ci-dessus:

– dichlorhydrate de 2-[2-[2-[4-[(4-chlorophényl)phénylméthyl]-1-pipérazinyl]éthoxy]éthoxy]-acétamide.
Le produit recristallise dans l'alcool isopropylique avec une molécule de solvant.

Rendement: 11 % P.F.: 100–102°C.

Analyse pour $C_{23}H_{30}ClN_3O_3 \cdot C_3H_7OH \cdot 2HCl$ en %

| | | | | | |
|---|---|---|---|---|---|
| calculé: | C 55,27 | H 7,16 | N 7,43 | $Cl^-$ 12,55 | $Cl^{tot.}$ 18,22 |
| trouvé: | C 53,10 | H 6,93 | N 7,18 | $Cl^-$ 12,56 | $Cl^{tot.}$ 18,79 |

1.3 2-[2-[4-[(4-chlorophényl)phénylméthyl]-1-pipérazinyl]éthoxy]-acétamide. (méthode I. 3.)

On dissout 2,3 g (0,0057 mole) de 2-[2-[4-[(4-chlorophényl)phénylméthyl]-1-pipérazinyl]éthoxy]-acétate de méthyle dans 100 ml de méthanol anhydre. On refroidit cette solution à une température de 5 à 10°C et on y fait barboter de l'ammoniac pendant 20 heures. On évapore le solvant sous vide, on triture le résidu dans de l'éther et on laisse cristalliser. On recueille 1,2 g de 2-[2-[4-[(4-chlorophényl)phénylméthyl]-1-pipérazinyl]éthoxy]-acétamide.

Rendement: 54% P.F.: 109–110°C.

Analyse pour $C_{21}H_{26}ClN_3O_2$ en %

| | | | | |
|---|---|---|---|---|
| calculé: | C 65,02 | H 6,71 | N 10,83 | Cl 9,14 |
| trouvé: | C 65,13 | H 6,59 | N 10,95 | Cl 9,54 |

Le 2-[2-[4-[(4-chlorophényl)phénylméthyl]-1-pipérazinyl]éthoxy]-acétate de méthyle utilisé comme produit de départ a été préparé comme suit:
On chauffe à reflux pendant 40 heures, sous bonne agitation, un mélange de 87 g (0,30 mole) de 1-[(4-chlorophényl)phénylméthyl]-pipérazine, de 58 g (0,38 mole) de (2-chloroéthoxy)-acétate de méthyle et de 40,3 g (0,38 mole) de carbonate de sodium dans 500 ml de xylène anhydre.
On refroidit, on filtre et on lave le solide avec du benzène et on écarte le solide lavé. Le filtrat est évaporé à sec. On purifie le résidu par chromatographie sur colonne de silice (éluant: chloroforme-méthanol, 97 : 3). On obtient ainsi 34 g (rendement: 27,8%) de l'ester méthylique souhaité.

Analyse pour $C_{22}H_{27}ClN_2O_3$ en %

| | | | |
|---|---|---|---|
| calculé: | C 65,60 | H 6,70 | N 6,95 |
| trouvé: | C 63,87 | H 6,55 | N 6,59 |

Deux sels d'addition de cet ester ont également été préparés:
– le dichlorhydrate: P.F.: 123–125°C.

Analyse pour $C_{22}H_{27}ClN_2O_3 \cdot 2HCl$ en %

| | | | | |
|---|---|---|---|---|
| calculé: | C 55,50 | H 6,10 | N 5,89 | $Cl^-$ 14,92 |
| trouvé: | C 55,20 | H 6,23 | N 5,65 | $Cl^-$ 13,2 |

– le dimaléate: P.F.: 128–130°C.

Analyse pour $C_{30}H_{35}ClN_2O_{11}$ en %

| | | | |
|---|---|---|---|
| calculé: | C 56,70 | H 5,51 | N 4,41 |
| trouvé: | C 57,01 | H 5,22 | N 4,45 |

Exemple 2. Préparation des acides de formule I ( Y = OH)

2.1 Acide 2-[2-[4-[(4-chlorophényl)phénylméthyl]-1-pipérazinyl]éthoxy]-acétique. (methode II)

On dissout 16,8 (0,0417 mole) de 2-[2-[4-[(4-chlorophényl)phénylméthyl]-1-pipérazinyl]éthoxy]-acétate de méthyle (préparé comme décrit à l'exemple 1.3 ci-dessus) dans 65 ml d'éthanol absolu. On y ajoute 42 ml d'une solution éthanolique d'hydroxyde de potassium 1 N. On chauffe le mélange à reflux pendant 4 heures. On refroidit et on élimine le précipité par filtration, après lavage à l'éther éthylique. Le filtrat est évaporé à sec. On triture le résidu dans de l'éther éthylique et on laisse cristalliser. On obtient 10,5 g de 2-[2-[4-[(4-chlorophényl)phénylméthyl)-1-pipérazinyl]éthoxy]-acétate de potassium (hygroscopique).

Rendement: 59% P.F.: 161–163°C.

Analyse pour $C_{21}H_{24}ClN_2O_3K$ en %

| | | | |
|---|---|---|---|
| calculé: | C 59,0 | H 5,63 | N 6,56 |
| trouvé: | C 57,97 | H 5,77 | N 6,48 |

On dissout le sel potassique dans 100 ml d'eau et on neutralise avec de l'acide chlorhydrique à 10% jusqu'à pH = 4. On extrait la solution au chloroforme et on sèche la phase organique sur du sulfate de magnésium. On évapore à sec. On triture le résidu dans de l'éther éthylique et on laisse cristalliser. On obtient 7,5 g l'acide 2-[2-[4-[(4-chlorophényl)phénylméthyl]-1-pipérazinyl]éthoxy]-acétique.

Rendement: 81% P.F.: 110–115°C.

Analyse pour $C_{21}H_{25}ClN_2O_3$ en %

| | | | |
|---|---|---|---|
| calculé: | C 64,80 | H 6,48 | N 7,20 |

trouvé: C 62,3 H 6,48 N 6,92

Le dichlorhydrate correspondant, préparé dans le toluène avec un rendement de 80%, fond à 225°C.

Analyse pour $C_{21}H_{25}ClN_2O_3 \cdot 2HCl$ en %

| | | | | | |
|---|---|---|---|---|---|
| calculé: | C 54,60 | H 5,85 | N 6,07 | $Cl^-$ 15,38 | $Cl^{tot.}$ 23,07 |
| trouvé: | C 54,42 | H 5,60 | N 6,01 | $Cl^-$ 15,29 | $Cl^{tot.}$ 23,08 |

2.2. Acide 2-[2-[4-(diphénylméthyl)-1-pipérazinyl]éthoxy]-acétique. (méthode II)

On chauffe à reflux, pendant 3 heures, un mélange de 19 g (0,054 mole) de 2-[2-[4-(diphénylméthyl)-1-pipérazinyl]éthoxy]-acétamide (obtenu comme décrit à l'exemple 1.1) dans 200 ml d'éthanol et de 27 ml d'une solution éthanolique d'hydroxyde de sodium 4 N. On neutralise avec 29,7 ml de'acide chlorhydrique (3,61 N) jusqu'à pH 6,3. On évapore l'éthanol sous vide. On filtre le précipité. Après l'évaporation du solvant, on obtient 17,4 g d'acide 2-[2-[4-(diphénylméthyl)-1-pipérazinyl]éthoxy]-acétique brut.

Rendement: 91 % P.F.: 100°C.

Analyse pour $C_{21}H_{26}N_2O_3$ en %

| | | | |
|---|---|---|---|
| calculé: | C 71,1 | H 7,39 | N 7,90 |
| trouvé: | C 69,1 | H 7,07 | N 7,12 |

Dichlorhydrate: P.F.: 217–218°C (alcool isopropylique)

Analyse pour $C_{21}H_{26}N_2O_3 \cdot 2HCl$ en %

| | | | | |
|---|---|---|---|---|
| calculé: | C 59,02 | H 6,60 | N 6,55 | $Cl^-$ 16,59 |
| trouvé: | C 58,83 | H 6,94 | N 6,33 | $Cl^-$ 15,90 |

Les produits suivants ont été préparés par la méthode décrite ci-dessus:

– dichlorhydrate de l'acide 2-[2-[2-[4-(diphénylméthyl)-1-pipérazinyl]éthoxy]éthoxy]-acétique.

Rendement: 57 % P.F.: 85°C (lyophylisé; décomposition)

Analyse pour $C_{23}H_{30}N_2O_4 \cdot 2HCl$ en %

| | | | | |
|---|---|---|---|---|
| calculé: | C 58,60 | H 6,84 | N 5,94 | $Cl^-$ 15,04 |
| trouvé: | C 56,82 | H 7,82 | N 6,02 | $Cl^-$ 16,76 |

– dichlorhydrate de l'acide 2-[2-[2-[4-[(4-chlorophényl)phénylméthyl]-1-pipérazinyl]éthoxy]-éthoxy]-acétique.

Rendement: 82 % P.F.: 112°C (lyophylisé).

Analyse pour $C_{23}H_{29}ClN_2O_4 \cdot 2HCl$ en %

| | | | | |
|---|---|---|---|---|
| calculé: | C 54,6 | H 5,78 | N 5,54 | $Cl^{tot.}$ 21,03 |
| trouvé: | C 52,48 | H 6,10 | N 5,72 | $Cl^{tot.}$ 22,19 |

– hydrate de l'acide 2-[2-[4-[(4-fluorophényl)phénylméthyl]-1-pipérazinyl]éthoxy]-acétique.

Rendement: 100 % P.F.: peu net (le produit se ramollit progressivement à partir de 70°C )

Analyse pour $C_{21}H_{25}FN_2O_3 \cdot 3/2H_2O$ en %

| | | | |
|---|---|---|---|
| calculé: | C 63,1 | H 7,0 | N 7,0 |
| trouvé: | C 63,7 | H 7,6 | N 6,9 |

– dichlorhydrate de l'acide 2-[2-[4-[(4-méthoxyphényl)phénylméthyl]-1-pipérazinyl]éthoxy]-acétique.

Rendement: 35 % P.F.: 214–217°C (acétonitrile; décomposition)

Analyse pour $C_{22}H_{28}N_2O_4 \cdot 2HCl$ en %

| | | | | |
|---|---|---|---|---|
| calculé: | C 57,7 | H 6,6 | N 6,1 | $Cl^-$ 15,5 |

trouvé: C 53,2 H 6,5 N 6,0 $Cl^-$ 17,5

– acide 2-[2-[4-[(2-chlorophényl)phénylméthyl]-1-pipérazinyl]éthoxy]-acétique.

Rendement: 50% P.F.: 96–100°C (lyophylisé).

Analyse pour $C_{21}H_{25}ClN_2O_3$ en %

| | | | | |
|---|---|---|---|---|
| calculé: | C 64,8 | H 6,5 | N 7,2 | Cl 9,1 |
| trouvé: | C 62,3 | H 6,9 | N 6,9 | Cl 10,2 |

– acide 2-[2-[2-[4-[[4-(trifluorométhyl)phényl]phénylméthyl]-1-pipérazinyl]éthoxy]éthoxy]-acétique.
– acide 2-[2-[2-[4-[bis(4-fluorophényl)méthyl]-1-pipérazinyl]éthoxy]éthoxy]-acétique.

B. Pharmacologie

Les produits suivants de la présente invention ont été soumis à des essais pharmacologiques dont les résultats sont reproduite ci-après:
– dichlorhydrate de 2-[2-[4-(diphénylméthyl)-1-pipérazinyl]éthoxy]-acétamide (produit A, préparé à l'exemple 1.1);
– 2-[2-[4-[(4-chlorophényl)phénylméthyl]-1-pipérazinyl]éthoxy]-acétamide (produit B, préparé aux exemples 1.1 et 1.3);
– 2-[2-[4-[(4-fluorophényl)phénylméthyl]-1-pipérazinyl]éthoxy]-acétamide (produit C, préparé à l'exemple 1.1);
– dichlorhydrate de 2-[2-[4-[(2-chlorophényl)phénylméthyl]-1-pipérazinyl]éthoxy]-acétamide (produit D, préparé à l'exemple 1.1);
– dichlorhydrate de 2-[2-[4-[(4-méthoxyphényl)phénylméthyl]-1-pipérazinyl]éthoxy]-acétamide (produit E, préparé à l'exemple 1.1);
– dichlorhydrate de 2-[2-[2-[4-(diphénylméthyl)-1-pipérazinyl]éthoxy]éthoxy]-acétamide (produit F, préparé à l'exemple 1.2);
– dichlorhydrate de 2-[2-[2-[4-[(4-chlorophényl)phénylméthyl]-1-pipérazinyl]éthoxy] éthoxy]-acétamide (produit G, préparé à l'exemple 1.2);
– 2-[2-[4-[(4-chlorophényl)phénylméthyl]-1-pipérazinyl]éthoxy]-acétate de potassium (produit H, préparé à l'exemple 2.1);
– acide 2-[2-[4-[(4-chlorophényl)phénylméthyl]-1-pipérazinyl]éthoxy]-acétique (produit I, préparé à l'exemple 2.1);
– dichlorhydrate de l'acide 2-[2-[4-[(4-chlorophényl)phénylméthyl]-1-pipérazinyl]éthoxy]-acétique (produit J, préparé à l'exemple 2.1);
– acide 2-[2-[4-(diphénylméthyl)-1-pipérazinyl]éthoxy]-acétique (produit K, préparé à l'exemple 2.2);
– dichlorhydrate de l'acide 2-[2-[2-[4-(diphénylméthyl)-1-pipérazinyl]éthoxy]éthoxy]-acétique (produit L, préparé à l'exemple 2.2);
– dichlorhydrate de l'acide 2-[2-[2-[4-[(4-chlorophényl)phénylméthyl]-1-pipérazinyl]éthoxy]éthoxy]-acétique (produit M, préparé à l'exemple 2.2);
– hydrate de l'acide 2-[2-[4-[(4-fluorophényl)phénylméthyl]-1-pipérazinyl]éthoxy]-acétique (produit N, préparé à l'exemple 2.2);

Les produits suivants qui ne font pas partie de l'invention (formule générale I dans laquelle m = 0) ont été soumis aux mêmes essais pharmacologiques:
– 2-[4-(diphénylméthyl)-1-pipérazinyl]-acétamide (produit 1). Ce produit a été préparé selon la méthode décrite dans le brevet belge 763.609. P.F.: 204°C.
– 2-[4-[(4-chlorophényl)phénylméthyl]-1-pipérazinyl]-acétamide (produit 2: voir H. B. WRIGHT et D. L. MARTIN, loc.cit.). Ce produit a également été préparé par la méthode décrite dans le brevet belge 763.609. P.F.: 145°C
– acide 2-[4-(diphénylméthyl)-1-pipérazinyl]-acétique (produit 3). Cette substance a été préparée par la méthode II (exemple 2.2). P.F.: 176°C.
– acide 2-[4-[(4-chlorophényl)phénylméthyl]-1-pipérazinyl]-acétique (produit 4). Cette substance a également été préparée par la méthode II (exemple 2.2). P.F: 106–108°C.

1. Activité antiallergique

Cette activité a été déterminée, chez le rat, au moyen du test »Passive Cutaneous Anaphylaxis (PCA)« (voir J. GOOSE et A. M. J. N. BLAIR, Immunology, 16, (1969), 749–60 et U. MARTIN et D. ROEMER, Arzneimittel-Forschung, 28, (1978) (5), 770–782).

On utilise des rats femelles dont les flancs ont été partiellement rasés. Dans la zone ainsi rasée, on injecte par voie intradermique, pour sensibiliser passivement les animaux, 0,05 ml de sérum »IGE antiovalbumine« à une dilution telle que, lors du test PCA, une tache nette d'une superficie d'environ 100 $mm^2$ apparaît à l'endroit de l'injection.

72 heures après l'injection, on administre, par voie intraveineuse, 0,25 ml d'une solution d'allergène renfermant un colorant (5 mg d'ovalbumine et 6 mg de Bleu Evans dans 0,25 ml d'une solution de chlorure de sodium à 0,9 %). A l'endroit de l'injection intradermique, on voit apparaître une tache bleue nette, dont on mesure la surface.

Pour tester l'activité des produits de l'invention, on opère de la même manière; toutefois

- le produit à tester est administré, per os, 72 heures après l'injection du sérum;
- minutes après cette administration, on injecte, par voie intraveineuse, 0,25 ml de la solution d'allergène;
- 30 minutes après l'administration de l'allergène, on mesure la surface de la tache bleue.

Dans le tableau ci-dessous, on reproduit les doses immunologiques (DI 50 en μmol/kg) qui provoquent, en moyenne sur l'ensemble des animaux soumis au test, une réduction 50 % de la superficie de la tache colorée.

De ce tableau, il ressort que, contrairement au cromoglycate de sodium, produit dont l'activité antiastmatique par voie intraveineuse est bien connue, les produits de l'invention sont actifs per os. Par contre, les produits 1, 2, 3 et 4 (non conformes à l'invention; m = O) s'avèrent peu intéressants.

| Produits | DI 50 per os μmol/kg |
|---|---|
| Cromoglycate de sodium | inactif |
| B | 36,5 |
| C | 18,9 |
| F | 10,6 |
| N | 10,2 |
| 1 | > 320 |
| 2 | > 320 |
| 3 | > 320 |
| 4 | > 320 |

2. Activité spasmolytique et antihistaminique

Ces activités ont été mesurées, chez le cobaye, par la méthode de H. KONZETT et R. ROESSLER (Naunyn-Schmiedebergs Arch. exp. Path. Pharmakol. 195, (1940), 71–74) et comparées à celle de la théophylline.

Le cobaye anesthésié et curarisé est ventilé artificiellement. La pression endotrachéale est enregistrée. Des spasmes bronchiques répétés sont induits par des injections intraveineuses successives et progressives, respectivement de sérotonine et d'histamine.

Les substances à tester sont également administrées par voie intraveineuse.

Dans le tableau ci-dessous figurent les doses de produits (DI 50 en μmol/kg) qui inhibent de 50 %, en moyenne sur l'ensemble des animaux, les bronchospasmes induits:

| Produits | Sérotonine | Histamine |
|---|---|---|
| Théophylline | 10 | 10 |
| A | 0,78 | 0,23 |
| B | 0,88 | 0,45 |
| C | 0,94 | 0,71 |
| D | 1,1 | 0,63 |
| E | 10,0 | 0,67 |
| F | 0,32 | 0,25 |
| G | 0,66 | 1,14 |
| H | 7,0 | 0,23 |
| I | 9,44 | 0,205 |
| J | 7,3 | 0,20 |
| K | 9,5 | 0,093 |
| L | 5,69 | 0,39 |
| M | 10,0 | 0,79 |
| N | 2,1 | 0,08 |
| 1 | 77 | 2,1 |
| 2 | 11 | 3,1 |
| 3 | > 32 | 5 |
| 4 | > 32 | 7,8 |

Les produits de l'invention sont dépourvus d'activité cholinergique. Il ressort de ce tableau que les produits de l'invention ont une activité remarquable à l'égard des bronchospasmes induits respectivement par la sérotonine et l'histamine, avec une sélectivité plus marquée envers cette dernière. Par contre, les produits 1, 2, 3 et 4 (non conformes à l'invention; m = 0) s'avèrent beaucoup moins actifs.

Par ailleurs, ce test a mis en évidence que certains composés, administrés à une dose unique, possèdent une activité antihistaminique de longue durée. Ainsi, par exemple, le produit J, administré au cobaye à la dose de 1 μmol/kg par voie intraveineuse conserve encore une activité égale à 100 % après 5 heures.

3. Comportement général de la souris (Test d'Irwin).

Ce comportement a été étudié par le test d'IRWIN (S. IRWIN, »General philosophy and methodology of screening: a multidimensional approach«, Gordon Research Conference on Medicinal Chemistry, Aug. 3–7 (1959) at Colby Junior College – New London).

Des doses progressives du produit soumis au test sont administrées, par voie intrapéritonéale, à des groupes de trois souris mâles (pesant de 18 à 22 g7 et le comportement général des animaux est observé selon les critères classiques.

Comme substances de référence, on utilise les produits suivants:

– Hydroxyzine: 2-[2-[4-[(4-chlorophényl)phénylméthyl]-1-pipérazinyl]éthoxy]-éthanol;
– Oxazepam: 7-chloro-1,3-dihydro-3-hydroxy-5-phényl-2H-1,4-benzodiazépine-2-one.

Dans le tableau di-dessous, on donne les doses (en mg/kg) qui induisent les premières manifestations de tranquillisation chez les animaux.

| Produits | Dose tranquillisante en mg/kg |
|---|---|
| A | > 255 |
| B | 38,7 |
| C | 115 |
| D | > 460 |
| E | 136 |
| F | 94 |
| G | 34 |
| H | 46,2 |
| J | 138 |
| K | 106 |
| L | 141 |
| M | 505 |
| N | 372 |
| Hydroxyzine | 27 |
| Oxazepam | 2,6 |

Il ressort de ce tableau que les produits de l'invention ont peu d'effet sédatif comparativement aux substances de référence.

Par ailleurs, dans ce test, la toxicité des produits de l'invention apparaît comme étant très faible.

Dans le tableau ci-dessous, on donne la dose mortelle (deux animaux sur trois) pour les produits de l'invention qui ont été administrés par voie intrapéritonéale chez la souris:

| Produits | Dose mortelle (en mg/kg) |
|---|---|
| A | 255 |
| B | 232 |
| C | 386 |
| D | 460 |
| E | 456 |
| F | 282 |
| G | 339 |
| H | 277 |
| I | 116 |
| J | 138 |
| K | 708 |
| L | 942 |
| M | 505 |
| N | 372 |

Lorsqu'on compare les deux tableaux (dose tranquillisante et dose mortelle) on constate que, pour certains produits l'effet sédatif n'apparaît qu'à des doses voisines de la dose mortelle.

4. Dose léthale (DL 50)

La faible toxicité des produits de l'invention a été confirmée par la mesure de la DL 50 per os.

Ainsi pour le produit J chez le rat Wistar, elle est de 703 mg/kg pour le rat mâle et de 865 mg/kg pour le rat femelle.

Chez la souris, pour le même produit, les DL 50 sont respectivement de 600 mg/kg (souris mâle) et de 752 mg/kg (souris femelle).

5. Posologie et administration

Les compositions pharmaceutiques contenant les composés de l'invention peuvent être administrées par voie orale, parentérale ou rectale. Elles peuvent aussi être utilisées par instillation nasale (aérosols) ou sous forme d'onguents ou de crèmes.

Les compositions pharmaceutiques utilisables pour l'administration orale peuvent être solides ou liquides, par exemple sous forme de comprimés (enrobés ou non), pilules, dragées, capsules en gélatine, solutions, sirops, etc. De même, les compositions utilisables pour l'administration par voie parentérale, sont les formes pharmaceutiquement connues pour ce genre d'administration, par exemple des solutions, suspensions ou émulsions aqueuses ou huileuses.

Pour l'administration par voie rectale, les compositions contenant les composés de l'invention se présentent généralement sous forme de suppositoires.

Les formes pharmaceutiques telles que solutions injectables, suspensions injectables, comprimés, gouttes, suppositoires, etc., sont préparées selon les méthodes couramment utilisées par les pharmaciens. On mélange les composés de l'invention avec un véhicule solide ou liquide, non toxique, pharmaceutiquement acceptable et éventuellement avec un agent dispersant, un agent désintégrant, un agent stabilisant, etc. On peut y ajouter, le cas échéant, des préservatifs, de agents édulcorants, des agents colorants, etc.

Le pourcentage de produit actif dans les compositions pharmaceutiques peut varier dans des limites très larges, selon le patient et le mode d'administration, en particulier selon la fréquence d'administration.

Quant à la posologie, elle peut varier dans une gamme étendue de doses unitaires, par exemple de 0,5 à 250 mg de produit actif.

A titre d'exemple non limitatif d'une composition contenant les composés de l'invention, on donne ci-après un exemple de gélule utilisable per os:

| | |
|---|---|
| Produit J | 100 mg |
| lactose | 67 mg |
| stéarate de magnésium | 1 mg |
| dioxyde de silicium (aerosil) | 2 mg |

**Revendications pour les Etats contractans BE, CH, DE, FR, GB, IT, LI, LU, NL, SE**

1. Acides 2-[4-(diphénylméthyl)-1-pipérazinyl]-acétiques et leurs amides répondant à la formule générale

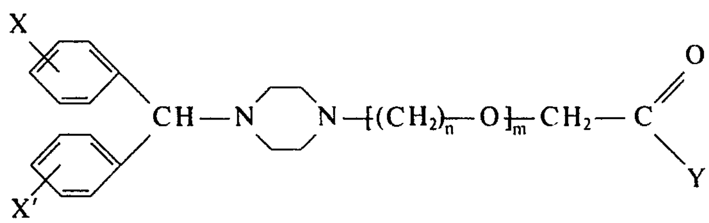

(I)

dans laquelle

Y est un groupe —OH ou un groupe —$NH_2$,
X et X', pris isolément, représentent un atome d'hydrogène, un atome d'halogène, un radical alkoxy linéaire ou ramifié en $C_1$—$C_4$ ou un radical trifluorométhyle,
m est égal à 1 ou 2,
n est égal à 1 ou 2,

ainsi que leurs sels non toxiques pharmaceutiquement acceptables.

2. Acide 2-[2-[4-[(4-chlorophényl)phénylméthyl]-1-pipérazinyl]éthoxy]-acétique et son dichlorhydrate.
3. Sel de potassium de l'acide 2-[2-[4-[(4-chlorophényl)phénylméthyl]-1-pipérazinyl]éthoxy]-acétique.
4. Acide 2-[2-[4-(diphénylméthyl)-1-pipérazinyl]éthoxy]-acétique et son dichlorhydrate.
5. Acide 2-[2-[4-[(4-fluorophényl)phénylméthyl]-1-pipérazinyl]éthoxy]-acétique et son hydrate.
6. Procédé de préparation d'acides 2-[4-(diphénylméthyl)-1-pipérazinyl]-acétiques, de leurs amides et de leurs sels tels que définis dans la revendication 1, caractérisé en ce que:

a) pour préparer les amides d'acides 2-[4-(diphénylméthyl)-1-pipérazinyl]-acétiques répondant à la formule générale (I) donnée à la revendication 1 et dans laquelle Y = $NH_2$, on fait réagir, dans un solvant inerte et en présence d'un accepteur d'acide, une 1-(diphénylméthyl)-pipérazine de formule

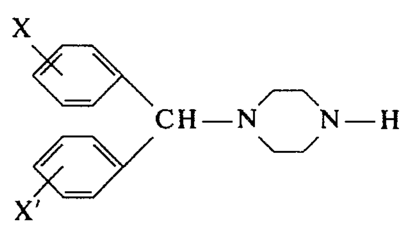

(II)

dans laquelle X et X' ont la signification donnée à la revendication 1, avec un oméga-halogénoacétamide de formule

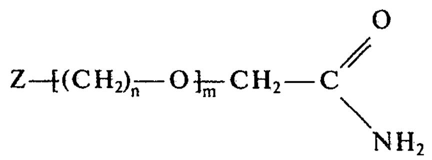

(III)

dans laquelle m et n ont la signification donnée à la revendication 1 et Z représente un atome d'halogène; ou

b) pour préparer les amides d'acides 2-[4-(diphénylméthyl)-1-pipérazinyl]-acétiques répondant à la formule générale (I) donnée à la revendication 1 et dans laquelle $Y = -NH_2$, on fait réagir, dans un solvant inerte, un sel de métal alcalin d'un oméga-[4-(diphénylméthyl)-1-pipérazinyl]-alcanol de formule

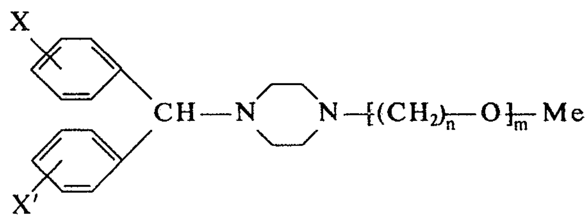

(IV)

dans laquelle X, X', m et n ont la signification donnée à la revendication 1 et Me représente un métal alcalin, avec un 2-halogénoacétamide de formule

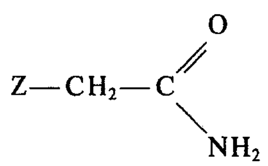

(V)

dans laquelle Z représente un atome d'halogène; ou

c) pour préparer les amides d'acides 2-[4-(diphénylméthyl)-1-pipérazinyl]-acétiques répondant à la formule générale (I) donnée à la revendication 1 et dans laquelle $Y = -NH_2$, on fait réagir, dans un solvant inerte, l'ammoniac (VI) avec un dérivé fonctionnel d'un acide 2-[4-(diphénylméthyl)-1-pipérazinyl]-acétique de formule

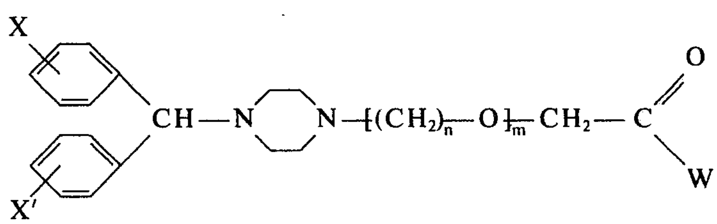

(VII)

dans laquelle X, X', m et n ont la signification donnée à la revendication 1 et W représente un atome d'halogène ou un groupe —OR' dans lequel R' représente un radical alkyle inférieur; ou

d) pour préparer les acides 2-[4-(diphénylméthyl)-1-pipérazinyl]-acétiques répondant à la formule générale (I) donnée à la revendication 1 et dans laquelle Y = OH, on hydrolyse, en milieu aqueux ou hydroalcoolique et par une base minérale, un dérivé fonctionnel d'un acide 2-[4-(diphénylméthyl)-1-pipérazinyl]-acétique de formule

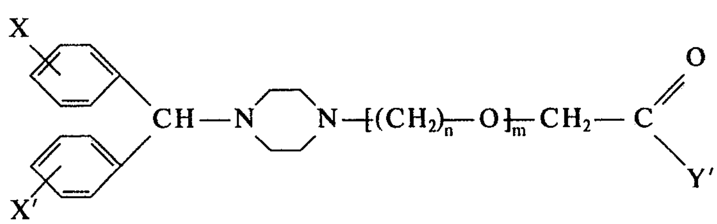

(VIII)

dans laquelle X, X', m et n ont la signification donnée à la revendication 1 et Y' représente un groupe $-NH_2$ ou un groupe —OR' dans lequel R' représente un radical alkyle inférieur; et

e) en ce que, éventuellement, on convertit les acides ou les amides d'acides 2-[4-(diphénylméthyl)-1-pipérazinyl]-acétiques ainsi obtenus en leurs sels non toxiques pharmaceutiquement acceptables.

7. Compositions thérapeutiques renfermant une quantité thérapeutiquement efficace d'un acide 2-[4-(diphénylméthyl)-1-pipérazinyl]-acétique ou de son amide selon la revendication 1 ou d'un sel non toxique pharmaceutiquement acceptable de ceux-ci, en association avec des excipients pharmaceutiques solides ou liquides.

**Revendication pour l'etat contractant: AT**

Procédé de préparation d'acides 2-[4-(diphénylméthyl)-1-pipérazinyl]-acétiques et de leurs amides répondant à la formule générale

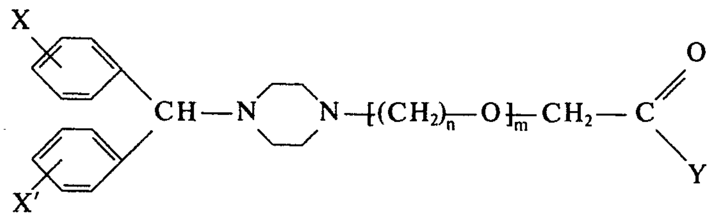

(I)

dans laquelle

Y est un groupe $-OH$ ou un groupe $-NH_2$,
X et X', pris isolément, représentent un atome d'hydrogène, un atome d'halogène, un radical alkoxy linéaire ou ramifié en C 1 à C 4 ou un radical trifluorométhyle,
m est égal à 1 ou 2,
n est égal à 1 ou 2,

ainsi que de leurs sels non toxiques pharmaceutiquement acceptables, caractérisé en ce que:

a) pour préparer les amides d'acides 2-[4-(diphénylméthyl)-1-pipérazinyl]-acétiques répondant à la formule générale (I) donnée ci-dessus et dans laquelle $Y = NH_2$, on fait réagir, dans un solvant inerte et en présence d'un accepteur d'acide, une 1-(diphénylméthyl)-pipérazine de formule

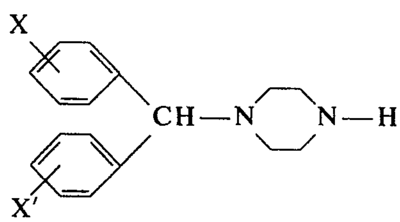

(II)

dans laquelle X et X' ont la signification donnée ci-dessus, avec un oméga-halogénoacétamide de formule

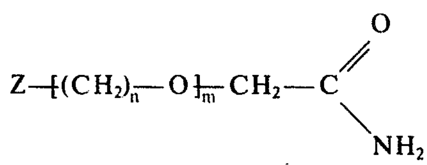

(III)

dans laquelle m et n ont la signification donnée ci-dessus et Z représente un atome d'halogène; ou

b) pour préparer les amides d'acides 2-[4-(diphénylméthyl)-1-pipérazinyl]-acétiques répondant à la formule générale (I) donnée ci-dessus et dans laquelle $Y = NH_2$, on fait réagir, dans un solvant inerte, un sel de métal alcalin d'un oméga-[4-(diphénylméthyl)-1-pipérazinyl]-alcanol de formule

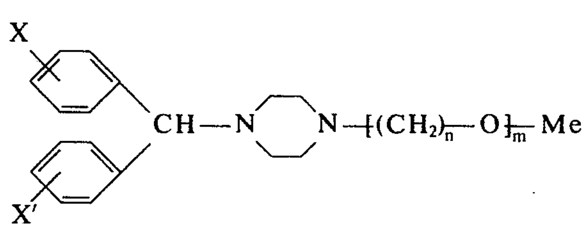

(IV)

dans laquelle X, X', m et n ont la signification donnée ci-dessus et Me représente un métal alcalin, avec un 2-halogénoacétamide de formule

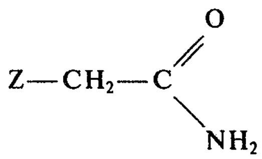

(V)

dans laquelle Z représente un atome d'halogène; ou

c) pour préparer les amides d'acides 2-[4-(diphénylméthyl)-1-pipérazinyl]-acétiques répondant à la formule générale (I) donnée ci-dessus et dans laquelle Y = $NH_2$, on fait réagir, dans un solvant inerte, l'ammoniac (VI) avec un dérivé fonctionnel d'un acide 2-[4-(diphénylméthyl)-1-pipérazinyl]-acétique de formule

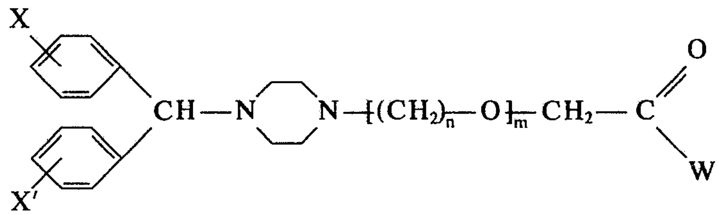

(VII)

dans laquelle X, X', m et n ont la signification donnée ci-dessus et W représente un atome d'halogène ou un groupe —OR' dans lequel R' représente un radical alkyle inférieur; ou

d) pour préparer les acides 2-[4-(diphénylméthyl)-1-pipérazinyl]-acétiques répondant à la formule générale (I) donnée ci-dessus et dans laquelle Y = OH, on hydrolyse, en milieu aqueux ou hydroalcoolique et par une base minérale, un dérivé fonctionnel d'un acide 2-[4-(diphénylméthyl)-1-pipérazinyl]-acétique de formule

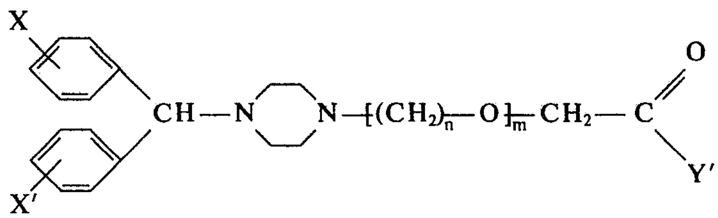

(VIII)

dans laquelle X, X', m et n ont la signification donnée ci-dessus et Y' représente un groupe $-NH_2$ ou un radical —OR' dans lequel R' représente un radical alkyle inférieur; et

e) en ce que, éventuellement, on convertit les acides ou les amides d'acides 2-[4-(diphénylméthyl)-1-pipérazinyl]-acétiques ainsi obtenus en leurs sels non toxiques pharmaceutiquement acceptables.

**Patentansprüche für die Vertragsstaaten: BE, CH, DE, FR, GB, IT, LI, LU, NL und SE**

1. 2-[4-(Diphenylmethyl)-1-piperazinyl]-essigsäuren und ihre Amide entsprechend der allgemeinen Formel

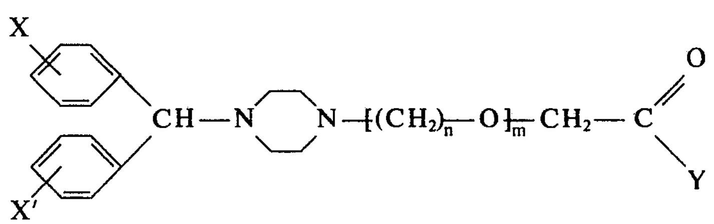

(I)

worin

Y ein —OH-Rest oder ein $-NH_2$-Rest ist,
X und X', einzeln genommen, ein Wasserstoffatom, ein Halogenatom, einen gerad- oder verzweigtkettigen $C_1$—$C_4$-Alkoxyrest oder einen Trifluormethylrest darstellt,
m 1 oder 2 ist,
n 1 oder 2 ist,

sowie deren nicht-toxische pharmazeutisch annehmbare Salze.

2. 2-[2-[4-[(4-Chlorphenyl)phenylmethyl]-1-piperazinyl]ethoxy]-essigsäure und ihr Dihydrochlorid.

3. Kaliumsalz der 2-[2-[4-[(4-Chlorphenyl)phenylmethyl]-1-piperazinyl]-ethoxy]-essigsäure.

4. 2-[2-[4-(Diphenylmethyl)-1-piperazinyl]ethoxy]-essigsäure und ihr Dihydrochlorid.

5. 2-[2-[4-[(4-Fluorphenyl)phenylmethyl]-1-piperazinyl]ethoxy]-essigsäure und ihr Hydrat.

6. Verfahren zur Herstellung von den in Anspruch 1 bestimmten 2-[4-(Diphenylmethyl)-1-piperazinyl]-essigsäuren, ihren Amiden und ihren Salzen, dadurch gekennzeichnet, daß man

a) um Amide von 2-[4-(Diphenylmethyl)-1-piperazinyl]-essigsäuren der in Anspruch 1 gegebenen allgemeinen Formel (I) und worin Y = $NH_2$ herzustellen, ein 1-(Diphenylmethyl)-piperazin der Formel

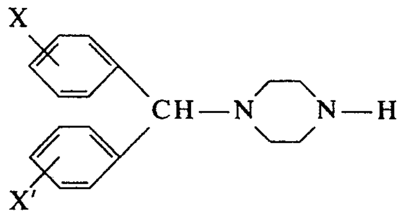

(II)

worin X und X' die in Anspruch 1 angegebene Bedeutung haben, mit einem omega-Haloacetamid der Formel

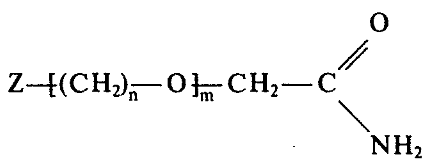

(III)

worin m und n die in Anspruch 1 angegebene Bedeutung haben und Z ein Halogenatom darstellt, in einem inerten Lösungsmittel und in Gegenwart eines Säureakzeptors, umsetzt; oder

b) um Amide von 2-[4-(Diphenylmethyl)-1-piperazinyl]-essigsäuren der in Anspruch 1 gegebenen allgemeinen Formel (I) und worin Y = $NH_2$ herzustellen, ein Alkalimetallsalz eines omega-[4-(Diphenylmethyl)-1-piperazinyl]-alkanols der Formel

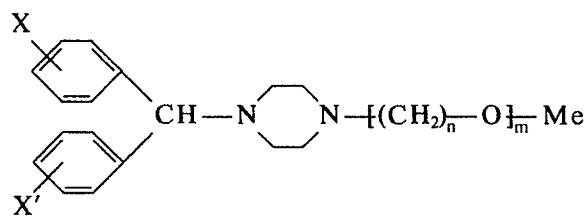

(IV)

worin X, X', m und n die in Anspruch 1 angegebene Bedeutung haben und Me ein Alkalimetall darstellt, mit einem 2-Haloacetamid der Formel

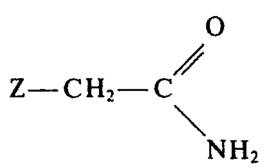

(V)

worin Z ein Halogenatom darstellt, in einem inerten Lösungsmittel, umsetzt; oder

c) um Amide von 2-[4-(Diphenylmethyl)-1-piperazinyl]-essigsäuren der in Anspruch 1 gegebenen allgemeinen Formel (I) und worin Y = $NH_2$ herzustellen, Ammoniak (VI) mit einem funktionellen Derivat einer 2-[4-(Diphenylmethyl)-1-piperazinyl]-essigsäure der Formel

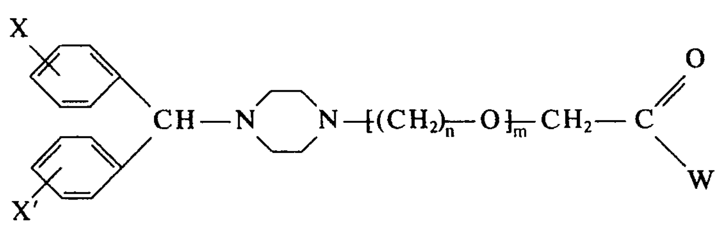

(VII)

worin X, X', m und n die in Anspruch 1 angegebene Bedeutung haben und W ein Halogenatom oder einen –OR'-Rest darstellt, worin R' einen niedrigen Alkylrest bedeutet, in einem inerten Lösungs-

mittel, umsetzt; oder

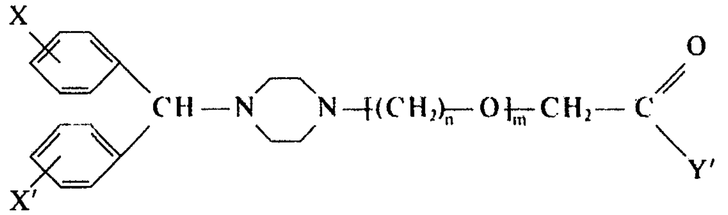

(VIII)

d) um 2-[4-(Diphenylmethyl)-1-piperazinyl]-essigsäuren der in Anspruch (I) gegebenen allgemeinen Formel (I) und worin Y = OH herzustellen, ein funktionelles Derivat einer 2-[4-(Diphenylmethyl)-1-piperazinyl]-essigsäure der Formel

worin X, X', m und n die in Anspruch 1 angegebene Bedeutung haben und Y' einen $-NH_2$-Rest oder einen —OR'-Rest darstellt, worin R' einen niedrigen Alkylrest bedeutet, in einem wäßrigen oder hydroalkoholischen Mittel und mit einer anorganischen Base, hydrolysiert; und

e) gegebenenfalls die so erhaltenen 2-[4-(Diphenylmethyl)-1-piperazinyl]-essigsäuren oder Amide in ihre nicht-toxischen pharmazeutisch annehmbaren Salze umwandelt.

7. Heilmittel, enthaltend eine therapeutisch wirksame Menge einer 2-[4-(Diphenylmethyl)-1-piperazinyl]-essigsäure oder ihres Amides gemäß Anspruch 1 oder eines nicht-toxischen pharmazeutisch annehmbaren Salzes davon in Verbindung mit festen oder flüssigen pharmazeutischen Hilfsstoffen.

**Patentanspruch für den Vertragsstaat: AT**

Verfahren zur Herstellung von 2-[4-(Diphenylmethyl)-1-piperazinyl]-essigsäuren und ihren Amiden entsprechend der allgemeinen Formel

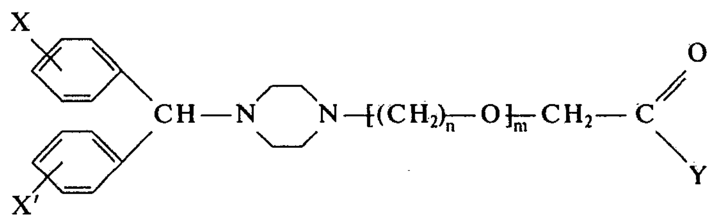

(I)

worin

Y ein —OH-Rest oder ein $-NH_2$-Rest ist,
X und X', einzeln genommen, ein Wasserstoffatom, ein Halogenatom, einen gerad- oder verzweigtkettigen $C_1$—$C_4$-Alkoxyrest oder einen Trifluormethylrest darstellt,
m 1 oder 2 ist,
n 1 oder 2 ist,

sowie von deren nicht toxischen pharmazeutisch annehmbaren Salzen, dadurch gekennzeichnet, daß man

a) um Amide von 2-[4-(Diphenylmethyl)-1-piperazinyl]-essigsäuren der oben angegebenen allgemeinen Formel (I) und worin Y = $NH_2$ herzustellen, ein 1-(Diphenylmethyl)-piperazin der Formel

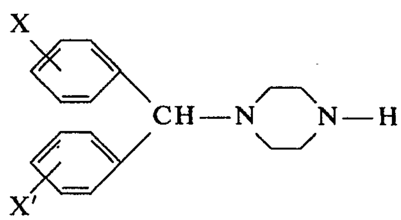

(II)

worin X und X' die oben angegebene Bedeutung haben, mit einem omega-Haloacetamid der Formel

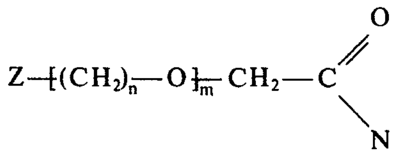

(III)

worin m und n die oben angegebene Bedeutung haben und Z ein Halogenatom darstellt, in einem inerten Lösungsmittel und in Gegenwart eines Säureakzeptors, umsetzt; oder

b) um Amide von 2-[4-(Diphenylmethyl)-1-piperazinyl]-essigsäuren der oben angegebenen allgemeinen Formel (I) und worin Y = $NH_2$ herzustellen, ein Alkalimetallsalz eines omega-[4-(Diphenylmethyl)-1-piperazinyl]-alkanols der Formel

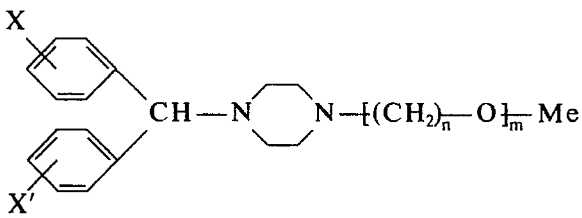

(IV)

worin X, X', m und n die oben angegebene Bedeutung haben und Me ein Alkalimetall darstellt, mit einem 2-Haloacetamid der Formel

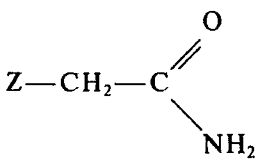

(V)

worin Z ein Halogenatom darstellt, in einem inerten Lösungsmittel, umsetzt; oder

c) um Amide von 2-[4-(Diphenylmethyl)-1-piperazinyl]-essigsäuren der oben angegebenen allgemeinen Formel (I) und worin Y = $NH_2$ herzustellen, Ammoniak (VI) mit einem funktionellen Derivat einer 2-[4-(Diphenylmethyl)-1-piperazinyl]-essigsäure der Formel

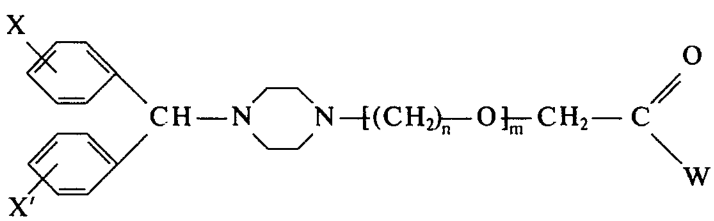

(VII)

worin X, X', m und n die oben angegebene Bedeutung haben und W ein Halogenatom oder einen —OR'-Rest darstellt, worin R' einen niedrigen Alkylrest bedeutet, in einem inerten Lösungsmittel, umsetzt; oder

d) um 2-[4-(Diphenylmethyl)-1-piperazinyl]-essigsäuren der oben angegebenen allgemeinen Formel (I) und worin Y = OH herzustellen, ein funktionelles Derivat einer 2-[4-(Diphenylmethyl)-1-piperazinyl]-essigsäure der Formel

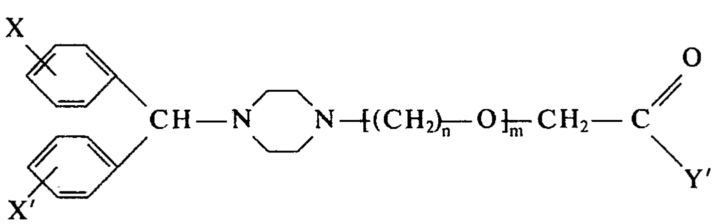

(VIII)

worin X, X', m und n die oben angegebene Bedeutung haben und Y' einen $-NH_2$-Rest oder einen —OR'-Rest darstellt, worin R' einen niedrigen Alkylrest bedeutet, in einem wäßrigen oder hydroalkoholischen Mittel und mit einer anorganischen Base, hydrolysiert; und

e) gegebenenfalls die so erhaltenen 2-[4-(Diphenylmethyl)-1-piperazinyl]-essigsäuren oder Amide in ihre nicht-toxischen pharmazeutisch annehmbaren Salze umwandelt.

**Claims for the Contracting States: BE, CH, DE, FR, GB, IT, LI, LU, NL and SE**

1. 2-[4-(diphenylmethyl)-1-piperazinyl]-acetic acids and their amides having the general formula

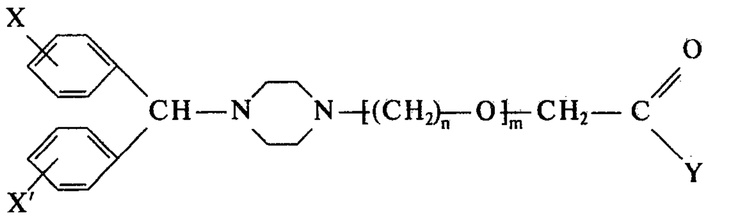

(I)

wherein

Y is an $-OH$ group or an $-NH_2$ group,

X and X', taken separately, represent a hydrogen atom, a halogen atom, a straight-chain or branched $C_1-C_4$-alkoxy radical or a trifluoromethyl radical,

m is 1 or 2,

n is 1 or 2,

as well as the non-toxic pharmaceutically acceptable salts thereof.

2. 2-[2-[4-[(4-Chlorophenyl)phenylmethyl]-1-piperazinyl]ethoxy]-acetic acid and its dihydrochloride.

3. Potassium salt of 2-[2-[4-[(4-chlorophenyl)phenylmethyl]-1-piperazinyl]ethoxy]-acetic acid.

4. 2-[2-[4-(Diphenylmethyl)-1-piperazinyl]ethoxy]-acetic acid and its dihydrochloride.

5. 2-[2-[4-[(4-Fluorophenyl)phenylmethyl]-1-piperazinyl]ethoxy]-acetic acid and its hydrate.

6. Process for the preparation of 2-[4-(diphenylmethyl)-1-piperazinyl]-acetic acids, their amides and their salts as defined in claim 1, characterised in that:

a) in order to prepare the amides of 2-[4-(diphenylmethyl)-1-piperazinyl]-acetic acids having the general formula (I) given in claim 1 and wherein $Y = NH_2$, a 1-(diphenylmethyl)-piperazinyl of the formula

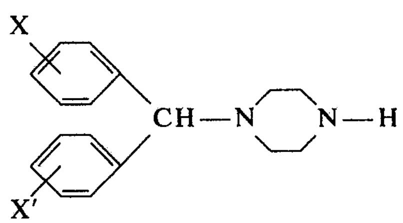

(II)

wherein X and X' have the meaning given in claim 1, is reacted, in an inert solvent and in the presence of an acid acceptor, with an omega-haloacetamide of the formula

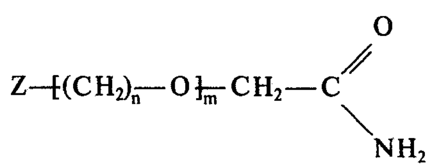

(III)

wherein m and n have the meaning given in claim 1 and Z represents a halogen atom; or

b) in order to prepare the amides of 2-[4-(diphenylmethyl)-1-piperazinyl]-acetic acids having the general formula (I) given in claim 1 and wherein $Y = NH_2$, an alkali metal salt of an omega-[4-(diphenylmethyl)piperazinyl]-alkanol of the formula

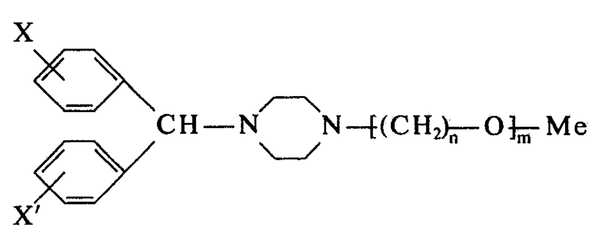

(IV)

wherein X, X' m and n have the meaning given in claim 1 and Me represents an alkali metal, is reacted, in an inert solvent, with a 2-haloacetamide of the formula

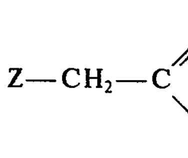

(V)

wherein Z represents a halogen atom; or

c) in order to prepare the amides of 2-[4-(diphenylmethyl)-1-piperazinyl]-acetic acids having the general formula (I) given in claim 1 and wherein $Y = NH_2$, ammonia (VI) is reacted, in an inert solvent, with a functional derivative of a 2-[4-(diphenylmethyl)-1-piperazinyl-acetic acids of the formula

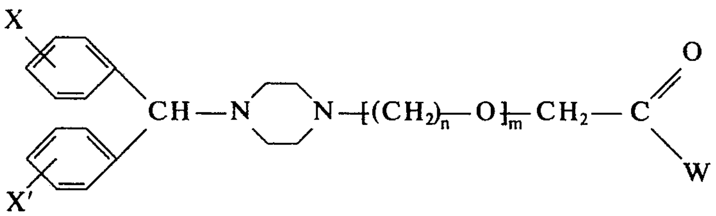

(VII)

wherein X, X′, m and n have the meaning given in claim 1 and W represents a halogen atom or an $-OR'$ group, wherein R′ represents a lower alkyl radical; or

d) in order to prepare the 2-[4-(diphenylmethyl)-1-piperazinyl]-acetic acids having the general formula (I) given in claim 1 and wherein $Y = OH$, a functional derivative of a 2-[4-(diphenylmethyl)-1-piperazinyl]-acetic acids of the formula

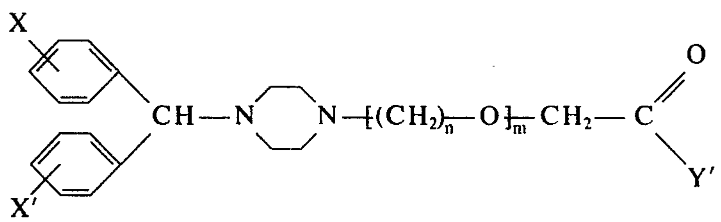

(VIII)

wherein X, X′, m and n have the meaning given in claim 1 and Y′ represents an $-NH_2$ group or an $-OR'$ group, wherein R′ represents a lower alkyl radical, is hydrolysed, in an aqueous or hydroalcoholic medium and with an inorganic base; and

e) optionally, the 2-[4-(diphenylmethyl)-1-piperazinyl]-acetic acids or amides thus obtained are converted into their nontoxic pharmaceutically acceptable salts.

7. Therapeutic compositions containing a therapeutically effective amount of a 2-[4-(diphenylmethyl)-1-piperazinyl]-acetic acids or its amide according to claim 1 or a non-toxic pharmaceutically acceptable salt thereof, in association with solid or liquid pharmaceutical excipients.

**Claim for the Contracting State: AT**

Process for the preparation of 2-[4-(diphenylmethyl)-1-piperazinyl]-acetic acids and their amides having the general formula

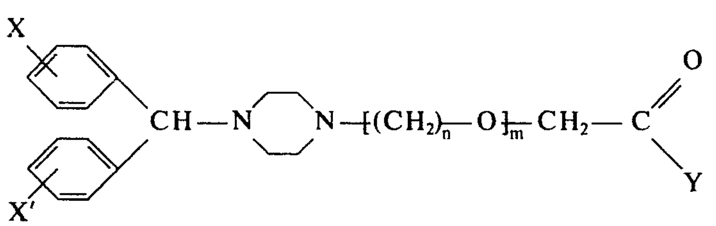

(I)

wherein

Y is an $-OH$ group or an $-NH_2$ group,

X and X′, taken separately, represent a hydrogen atom, a halogen atom, a straight-chain or branched $C_1-C_4$-alkoxy radical or a trifluoromethyl radical,

m is 1 or 2,

n is 1 or 2,

as well as the non-toxic pharmaceutically acceptable salts thereof, characterised in that:

a) in order to prepare the amides of 2-[4-(diphenylmethyl)-1-piperazinyl]-acetic acids having the general formula (I) given above and wherein Y = $NH_2$, a 1-(diphenylmethyl)-piperazinyl of the formula

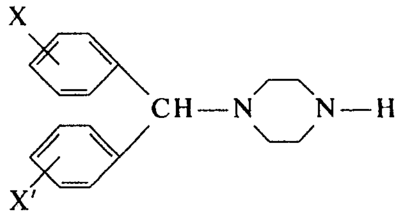

(II)

wherein X and X' have the meaning given above, is reacted, in an inert solvent and in the presence of an acid acceptor, with an omega-haloacetamide of the formula

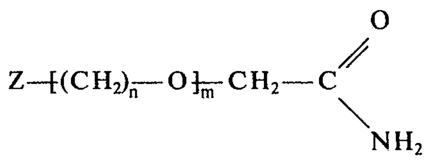

(III)

wherein m and n have the meaning given in claim 1 and Z represents a halogen atom; or

b) in order to prepare the amides of 2-[4-(diphenylmethyl)-1-piperazinyl]-acetic acids having the general formula (I) given above and wherein Y = $NH_2$, an alkali metal salt of an omega-[4-(diphenylmethyl)-1-piperazinyl]-alkanol of the formula

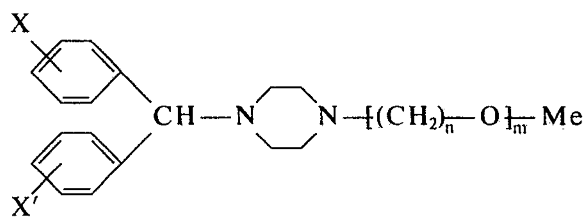

(IV)

wherein X, X' m and n have the meaning given above and Me represents an alkali metal, is reacted, in an inert solvent, with a 2-haloacetamide of the formula

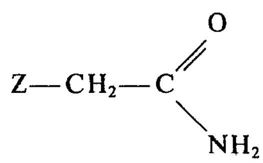

(V)

wherein Z represents a halogen atom; or

c) in order to prepare the amides of 2-[4-(diphenylmethyl)-1-piperazinyl]-acetic acids having the general formula (I) given above and wherein Y = $NH_2$, ammonia (VI) is reacted, in an inert solvent, with a functional derivative of a 2-[4-(diphenylmethyl)-1-piperazinyl-acetic acids of the formula

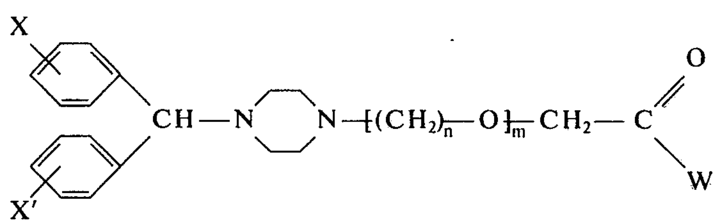

(VII)

wherein X, X', m and n have the meaning given above and W represents a halogen atom or an —OR' group, wherein R' represents a lower alkyl radical; or

d) in order to prepare the 2-[4-(diphenylmethyl)-1-piperazinyl]-acetic acids having the general formula (I) given above and wherein Y = OH, a functional derivative of a 2-[4-(diphenylmethyl)-1-piperazinyl]-acetic acids of the formula

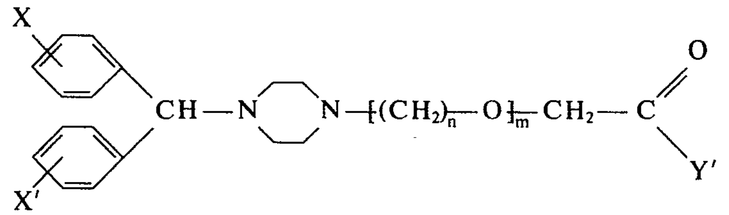

(VIII)

wherein X, X', m and n have the meaning given in claim 1 and Y' represents an $-NH_2$ group or an $-OR'$ radical, wherein R' represents a lower alkyl radical, is hydrolysed, in an aqueous or hydroalcoholic medium and with an inorganic base; and

e) optionally, the 2-[4-(diphenylmethyl)-1-piperazinyl]-acetic acids or amides thus obtained are converted into their non-toxic pharmaceutically acceptable salts.